# FASCICULE DE BREVET EUROPEEN

(11) **EP 0 940 150 B1**
(45) Date de publication et mention de la délivrance du brevet: **05.06.2002**
(21) Numéro de dépôt: 99400501.5
(22) Date de dépôt: 02.03.1999
(51) Int. Cl.: A61M 1/00

(54) **Dispositif d'aspiration des mucosités nasales**
Vorrichtung zum Absaugen von Nasenschleim
Nose mucus aspiration device

(30) Priorité: 03.03.1998 FR 9802537
(43) Date de publication de la demande: 08.09.1999
(73) Titulaire: Mougeolle, Philippe Louis Paul Edouard, 1941 Volleges (CH)
(72) Inventeur: Mougeolle, Philippe Louis Paul Edouard, 1941 Volleges (CH)
(74) Mandataire: Colas, Jean-Pierre

(56) Documents cités:
- EP-A- 0 451 062
- WO-A-92/00111
- CH-A- 687 851
- FR-A- 359 401

## Description

La présente invention est relative à un dispositif d'aspiration des mucosités nasales, destiné en particulier à moucher les enfants en bas âge.

Un enfant en bas âge n'est pas capable d'expirer fortement par le nez afin d'expulser les mucosités qui s'y trouvent. Pour éviter l'obturation des voies respiratoires nasales, l'intervention d'un adulte est nécessaire.

De manière connue, l'adulte peut avoir recours à un liquide physiologique et à un bâtonnet dont les extrémités sont enveloppées de coton (coton-tige).

Cette méthode présente deux inconvénients. Tout d'abord, le diamètre du coton-tige est souvent trop grand par rapport à celui des narines d'un enfant en bas âge. Ensuite, le coton-tige risque de blesser l'enfant s'il est introduit trop profondément à l'intérieur de la cavité nasale.

De manière connue également, l'adulte peut avoir recours à une simple poire munie d'un embout permettant d'aspirer les mucosités par la dépression créée dans la poire.

Cette méthode présente l'inconvénient d'être peu hygiénique. En effet, l'intérieur de la poire est en général difficile à nettoyer. En outre, cette méthode suppose que l'on comprime la poire en dehors des narines de l'enfant, afin d'éviter d'y refouler des mucosités déjà aspirées. Ceci augmente le nombre des manipulations, et donc le temps de l'opération.

De manière connue également, l'adulte peut avoir recours à un embout fixé sur un tube. Il place l'embout dans les narines de l'enfant et aspire les mucosités nasales par le tube avec la bouche.

Bien que le tube puisse avoir une longueur suffisante pour éviter que les mucosités n'atteignent la bouche de l'adulte, cette méthode est manifestement peu hygiénique.

De manière connue également (voir le brevet suisse n° 687 851 du demandeur, ainsi que la demande de brevet européen n° 451 062), l'adulte peut avoir recours à un dispositif d'aspiration des mucosités nasales du type à valves. Ce dispositif comprend un corps formant une cavité comportant deux ouvertures. L'une de ces ouvertures est en communication de fluide étanche avec un embout amovible de forme sensiblement conique destiné à pénétrer dans les narines de l'enfant. L'autre ouverture est en communication de fluide étanche avec une chambre de compression/décompression fabriquée dans un matériau souple et élastique pourvu de deux valves.

L'une de ces valves communique avec l'extérieur. Elle permet, lorsque l'on comprime la chambre de compression/décompression, de chasser l'air qui s'y trouve directement vers l'extérieur, sans passer par l'embout.

L'autre valve communique avec l'intérieur du corps du dispositif. Elle permet, lorsque l'on relâche la chambre de compression/décompression depuis un état comprimé, d'aspirer l'air à travers l'embout et, par là-même, les mucosités nasales.

Un filtre est interposé entre cette dernière valve et l'intérieur du corps du dispositif, de manière à éviter que les mucosités nasales ne pénètrent à l'intérieur de la chambre de compression/décompression.

Ce dispositif, bien qu'efficace et hygiénique, pose des problèmes de fabrication. En effet, on ne peut pas réaliser les valves de la chambre de compression/décompression en une seule opération de moulage.

Ces valves doivent être rapportées sur la chambre de compression/décompression déjà moulée, ce qui augmente le coût de fabrication du dispositif et risque par ailleurs de porter préjudice à sa longévité.

La présente invention a pour but de remédier à ces inconvénients.

On atteint ce but de l'invention, ainsi que d'autres qui apparaîtront à la lecture de ce qui va suivre, avec un dispositif d'aspiration des mucosités nasales, comprenant un corps formant une cavité comportant des première et deuxième extrémités ouvertes, un embout amovible et une chambre de compression/décompression en communication de fluide étanche avec respectivement lesdites première et deuxième extrémités ouvertes, dans lequel ledit corps comporte deux valves (51, 52) dont l'une (52) est une valve d'aspiration placée à l'intérieur dudit corps entre lesdites première et deuxième extrémités ouvertes de manière à ne permettre une circulation d'air que de ladite première extrémité ouverte vers ladite deuxième extrémité ouverte, et dont l'autre (51) est une valve d'échappement placée à la périphérie dudit corps, entre ladite valve d'aspiration et ladite deuxième extrémité ouverte, de manière à ne permettre une circulation d'air que de l'intérieur dudit corps vers l'extérieur.

Ainsi, contrairement à ce qui est enseigné par le brevet suisse n° 687 851 et par la demande de brevet européen n° 451 062, on reporte les valves sur le corps du dispositif. On s'affranchit de la sorte des difficultés liées à l'intégration de ces valves à la chambre de compression/décompression.

Grâce à ces caractéristiques, il devient possible de réaliser au moindre coût un dispositif d'aspiration des mucosités nasales efficace, hygiénique et robuste et de créer aisément des chambres de compression/décompression sous forme de figurines.

D'autres caractéristiques et avantages de la présente invention apparaîtront à la lecture de la description qui va suivre et à l'examen des dessins annexés donnés uniquement à titre d'exemple, et dans lesquels :
- la figure 1 est une vue en perspective éclatée du dispositif selon l'invention ;
- la figure 2 est une vue en coupe longitudinale du dispositif selon l'invention lorsque l'on comprime la chambre de compression/décompression;
- la figure 3 est une vue en coupe longitudinale du dispositif selon l'invention lorsque l'on relâche la chambre de compression/décompression.

Sur ces figures, des références numériques identiques représentent des organes ou éléments identiques ou analogues.

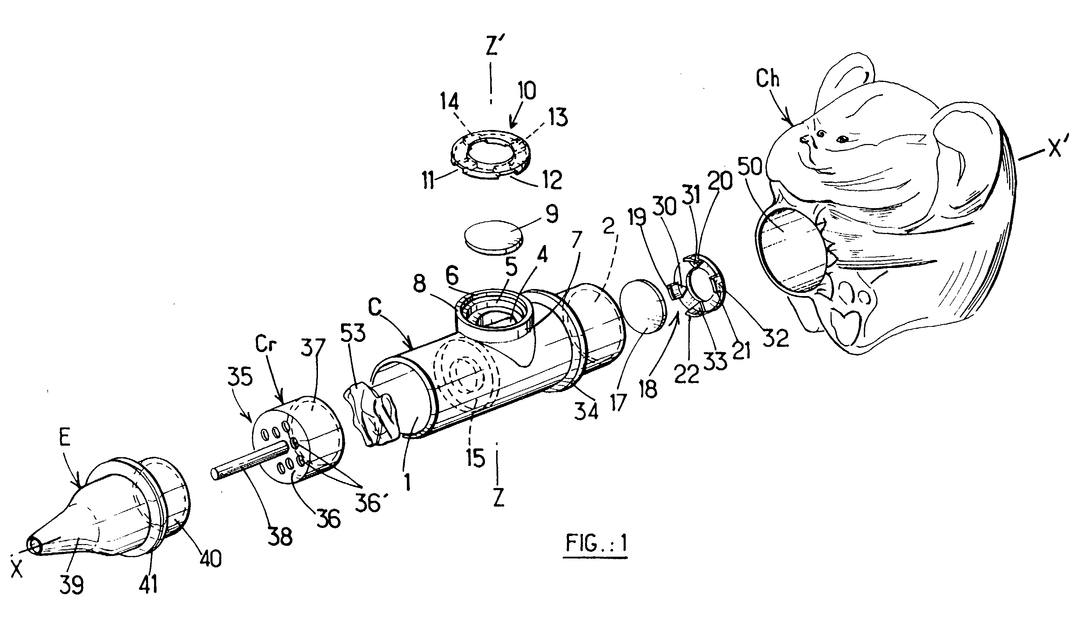

Sur la figure 1, on voit les différents organes constitutifs du dispositif selon l'invention. Ce dispositif comprend un corps C ayant par exemple la forme d'un cylindre creux d'axe XX', dont les première 1 et deuxième 2 extrémités sont ouvertes.

Ce corps C comporte, à sa périphérie et à peu près à mi-longueur, un orifice 4 de section par exemple circulaire surmonté de premier 5 et deuxième 6 alésages formés dans un appendice 7 de forme par exemple cylindrique. Ces deux éléments sont coaxiaux, et leur axe ZZ' coupe perpendiculairement l'axe XX' du corps C. L'appendice 7 se raccorde au corps C selon une géométrie classique d'intersection de deux cylindres d'axes concourants.

Une première pastille 9, de forme par exemple circulaire, est destinée à être posée au fond du premier alésage 5.

Une première pièce annulaire 10 est destinée à s'ajuster dans le deuxième alésage 6.

Cette première pièce annulaire comporte plusieurs rainures radiales, quatre par exemple 11,12,13,14, sur sa face destinée à venir en vis-à-vis de l'orifice 4.

Une collerette annulaire 15, d'axe confondu avec celui XX' du corps C, est fixée à l'intérieur de ce corps entre la première extrémité 1 et l'orifice 4. Cette collerette annulaire est de préférence située à proximité d'un plan P perpendiculaire à l'axe XX' et tangent à l'appendice 7.

Une deuxième pastille 17, de forme par exemple circulaire, est destinée à être posée sur la collerette annulaire 15 du côté situé en vis-à-vis de la deuxième extrémité 2 du corps C.

Une deuxième pièce annulaire 18 est destinée à être introduite par la deuxième extrémité 2 dans le tronçon 16 contre la collerette annulaire 15.

Cette deuxième pièce annulaire comporte plusieurs dents, quatre par exemple 19,20,21,22, sur la périphérie de sa face destinée à venir en vis-à-vis de la collerette annulaire 15.

Des petites saillies 30,31,32,33 situées sur cette face s'étendent radialement de la base des dents 19,20,21,22 vers le centre de cette deuxième pièce annulaire.

La hauteur hd des dents 19,20,21,22 est supérieure aux épaisseurs cumulées de la deuxième pastille 17 et des petites saillies 30,31,32,33.

Le corps C comporte par ailleurs sur sa périphérie extérieure, entre l'appendice 7 et la deuxième extrémité 2, un premier bourrelet annulaire 34.

Le dispositif selon l'invention comprend par ailleurs une crépine Cr. Cette crépine consiste en un cylindre creux dont une première extrémité 35 est fermée par un pan circulaire 36 pourvu d'une pluralité de petits orifices 36', et dont la deuxième extrémité 37 est ouverte.

Un bâtonnet d'extraction 38, de longueur à peu près égale à celle séparant la première extrémité 1 du corps C de la collerette annulaire 15, est fixé perpendiculairement au pan circulaire 36 en pointant vers l'extérieur de la crépine Cr.

Le dispositif selon l'invention comprend par ailleurs un embout amovible E. Dans le mode de réalisation représenté à titre d'exemple, cet embout comporte une partie sensiblement conique 39 dont la plus petite section peut entrer dans une narine d'enfant en bas âge.

La partie conique 39 est prolongée, du côté de sa plus grande section, par une partie cylindrique 40.

Un deuxième bourrelet annulaire 41 est situé sur la périphérie de l'embout E, à la frontière entre la partie conique 39 et la partie cylindrique 40.

Enfin, le dispositif selon l'invention comprend une chambre de compression/décompression Ch amovible réalisée dans un matériau souple et élastique. De préférence, cette chambre a la forme et les couleurs d'une figurine distrayante pour l'enfant. Sur la figure 1, on a par exemple représenté une figurine rappelant un petit éléphant.

La chambre de compression/décompression Ch comporte un embout cylindrique semi-rigide 50, moulé d'une même pièce avec la chambre.

Le corps C, les pièces annulaires 10 et 18, les pastilles 9 et 17, la crépine Cr et l'embout E peuvent être fabriqués en matière plastique. Le polycarbonate ou le polypropylène injectés conviennent particulièrement. Ces deux matériaux peuvent être soumis à une température atteignant 120°C, ce qui permet de nettoyer le dispositif selon l'invention par stérilisation.

La chambre de compression Ch peut être fabriquée en caoutchouc synthétique ou en tout autre matériau élastomère non toxique.

L'assemblage en usine du dispositif selon l'invention s'effectue de la manière suivante.

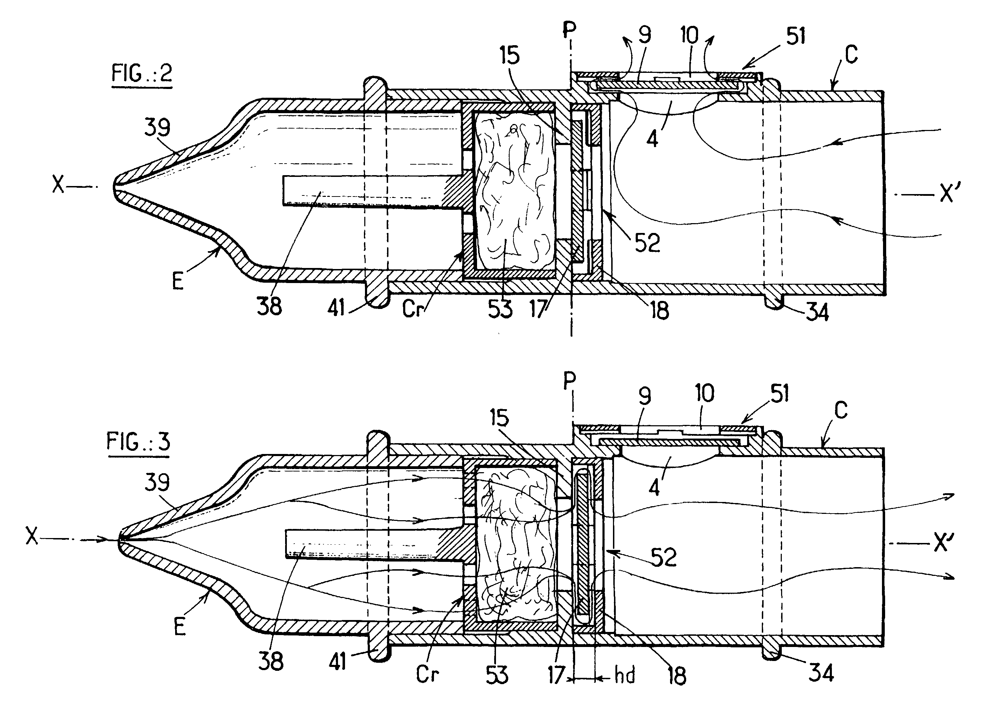

On pose la première pastille 9 au fond du premier alésage 5, et on ajuste par frottement la première pièce annulaire 10 dans le deuxième alésage 6, en prenant garde de placer les quatre rainures radiales 11,12,13,14 en vis-à-vis de l'orifice 4. On obtient ainsi un ensemble formant une valve d'échappement 51, visible sur les figures 2 et 3.

On introduit la deuxième pastille 17 dans le corps C par la deuxième extrémité ouverte 2, et on la pose sur la collerette annulaire 15. On introduit de la même manière la deuxième pièce annulaire 18 que l'on ajuste par frottement dans le corps C contre la collerette annulaire 15. On prend garde d'une part de placer les quatre dents 19,20,21,22 en vis-à-vis de cette collerette annulaire, et d'autre part d'insérer la deuxième pastille 17 entre ces quatre dents. On obtient ainsi un ensemble formant une valve d'aspiration 52, visible sur les figures 2 et 3.

Pour l'utilisation du dispositif selon l'invention, il suffit d'ajuster par frottement l'embout cylindrique semi-rigide 50 de la chambre de compression/décompression Ch sur la deuxième extrémité 2 du corps C.

On introduit, si on le souhaite, un matériau filtrant 53, tel que du coton, à l'intérieur de la crépine Cr par sa deuxième extrémité 37. On introduit la crépine éventuellement pourvue de son matériau filtrant en la tenant par le bâtonnet d'extraction 38 dans la première extrémité ouverte 1, et on l'ajuste par frottement dans le corps C contre la collerette annulaire 15.

Enfin, on ajuste par frottement l'embout amovible E dans la première extrémité 1 du corps C.

Comme on peut le voir, l'assemblage du dispositif selon l'invention est extrêmement simple, et ne nécessite en particulier aucun moyen de vissage, de collage, etc.

Les dimensions des pièces sont étudiées pour que l'étanchéité des liaisons soit assurée par de simples ajustements par frottement, ce qui est particulièrement commode pour l'utilisateur, lorsqu'après avoir nettoyé le dispositif, celui-ci est de nouveau remonté.

Comme on peut le voir également, la conception des valves d'échappement 51 et d'aspiration 52 est extrêmement simplifiée. On remarquera en particulier que ces valves sont totalement indépendantes de la chambre Ch, et donc que les problèmes de moulage de cette chambre rencontrés dans la technique antérieure sont ici résolus.

L'utilisation et le principe de fonctionnement du dispositif selon l'invention sont les suivants (voir figures 2 et 3).

On présente l'embout E face à une narine, et on l'y fait pénétrer jusqu'à ce qu'une communication de fluide étanche puisse s'établir entre la narine et l'embout.

On saisit le dispositif par la chambre de compression/décompression Ch avec une seule main, et on comprime cette chambre.

Comme cela est visible sur la figure 2, l'air chassé de la chambre pénètre dans le corps C, pousse la première pastille 9 contre la première pièce annulaire 10 et pousse la deuxième pastille 17 contre la collerette annulaire 15. Ceci a pour effet d'ouvrir la valve d'échappement 51, dans laquelle l'air s'échappe par les quatre rainures radiales 11,12,13,14, et de fermer la valve d'aspiration 52. On peut ainsi comprimer la chambre Ch sans envoyer d'air dans le nez de l'enfant.

On relâche ensuite lentement la chambre Ch, ce qui crée une dépression à l'intérieur du corps C (voir figure 3). Cette dépression a pour effet de plaquer la première pastille 9 contre le fond de l'alésage 5, et de plaquer la deuxième pastille 17 contre la deuxième pièce annulaire 18. Ceci entraîne la fermeture de la valve d'échappement 51 et l'ouverture de la valve d'aspiration 52, dans laquelle l'air circule à travers l'interstice ménagé par les quatre petite saillies 30,31,32,33 entre la deuxième pastille circulaire 17 et la deuxième pièce annulaire 18.

L'ouverture de la valve d'aspiration 52 a pour effet de mettre également en dépression la partie du corps C qui est comprise entre la collerette annulaire 15 et le pan circulaire 36 de la crépine Cr. Ce pan étant muni d'une pluralité de petits orifices 36', la dépression s'étend jusqu'à l'embout amovible E. Il en résulte une aspiration des mucosités nasales de l'enfant vers l'intérieur de l'embout E.

Ces mucosités peuvent, surtout si elles sont assez liquides, pénétrer jusque dans les petits orifices de la crépine Cr. Le matériau filtrant 53 éventuellement utilisé permet, tout en laissant passer l'air, d'empêcher que ces mucosités n'atteignent la valve d'aspiration 52, au risque de la boucher.

Si besoin est, on répète doucement plusieurs fois l'ensemble des opérations décrites ci-dessus pour chacune des narines de l'enfant. En général deux à trois compressions/décompressions par narine suffisent, ce qui représente une durée d'intervention très courte. Qui plus est, l'enfant est distrait par la forme et par les couleurs de la chambre de compression/décompression, de sorte qu'il ne s'impatiente pas.

Lorsque l'intervention est terminée, on sépare l'embout E du corps C en s'aidant des premier 34 et deuxième 41 bourrelets annulaires qui offrent des moyens de prise aisés.

On extrait la crépine Cr et, s'il y a lieu, le matériau filtrant 53 du corps C grâce au bâtonnet d'extraction 38 et on jette le matériau filtrant.

On sépare par ailleurs la chambre de compression/décompression Ch du corps C.

On lave ensuite l'embout E, la crépine Cr et le corps C, à l'eau chaude par exemple. Si besoin est, on peut stériliser l'ensemble de ces organes en les faisant bouillir.

Comme on peut le voir, le dispositif selon l'invention est très hygiénique, dans la mesure où son nettoyage est aisé. On notera en particulier que la chambre de compression/décompression Ch est complètement isolée de la partie du corps C dans laquelle peuvent se trouver des mucosités, et donc que cette chambre ne risque pas d'être souillée.

Bien entendu, le dispositif selon l'invention n'est pas limité au mode de réalisation décrit et représenté qui n'a été donné qu'à titre d'exemple. C'est ainsi par exemple que le corps C pourrait avoir une toute autre forme que celle d'un cylindre et que la chambre de compression/décompression Ch pourrait être remplacée par une autre figurine, par une simple poire, ou par tout autre mécanisme permettant de refouler l'air vers l'extérieur et de créer une dépression à l'intérieur du corps C. C'est ainsi également que les pièces annulaires 10 et 18 pourraient être identiques. Selon respectivement qu'elles auraient toutes les deux la forme de la pièce annulaire 10 ou bien celle de la pièce annulaire 18 décrites ci-dessus, l'alésage 5 serait supprimé, ou bien un alésage serait adjoint à la collerette annulaire 15. C'est ainsi également que l'embout E pourrait avoir toute autre forme ou pourrait être remplacé par un ensemble à deux embouts, de manière à aspirer simultanément les mucosités nasales dans les deux narines.

## Revendications

1. Dispositif d'aspiration des mucosités nasales, comprenant un corps (C) formant une cavité comportant des première (1) et deuxième (2) extrémités ouvertes, un embout amovible (E) et une chambre de compression/décompression (Ch) en communication de fluide étanche avec respectivement lesdites première et deuxième extrémités ouvertes, ledit corps comportant deux valves (51, 52) dont l'une (52) est une valve d'aspiration placée à l'intérieur dudit corps entre lesdites première et deuxième extrémités ouvertes de manière à ne permettre une circulation d'air que de ladite première extrémité ouverte vers ladite deuxième extrémité ouverte, et dont l'autre (51) est une valve d'échappement placée à la périphérie dudit corps, entre ladite valve d'aspiration et ladite deuxième extrémité ouverte, de manière à ne permettre une circulation d'air que de l'intérieur dudit corps vers l'extérieur.

2. Dispositif selon la revendication 1, **caractérisé en ce que** l'une ou l'autre des deux valves (51; 52) comprend une première pastille (9) posée sur un orifice (4) formé au fond d'un alésage (5) et surmontée d'une première pièce annulaire (10) comportant, sur sa face destinée à venir en vis-à-vis dudit orifice, plusieurs rainures radiales (11,12,13,14).

3. Dispositif selon la revendication 1 ou 2, **caractérisé en ce que** l'une ou l'autre desdites deux valves (51; 52) comprend une deuxième pastille (17) posée sur une collerette annulaire (15) et surmontée d'une deuxième pièce annulaire (18) comportant, à la périphérie de sa face destinée à venir en vis-à-vis de ladite collerette annulaire, plusieurs dents (19,20,21,22) à la base desquelles des petites saillies (30,31,32,33) s'étendent radialement vers le centre de ladite deuxième pièce annulaire.

4. Dispositif selon l'une quelconque des revendications précédentes, **caractérisé en ce qu'**une crépine (Cr), comportant une première extrémité (35) fermée par un pan (36) pourvu d'une pluralité de petits orifices (36') et une deuxième extrémité (37) ouverte, est placée dans ledit corps (C) entre ladite première ouverture (1) et ladite valve d'aspiration (52), ladite deuxième extrémité ouverte étant située en vis-à-vis de ladite valve d'aspiration.

5. Dispositif selon la revendication 4, **caractérisé en ce que** ladite crépine (Cr) comporte un bâtonnet d'extraction (38) fixé rigidement audit pan (36), et **en ce que** la longueur dudit bâtonnet est à peu près égale à celle séparant ladite première extrémité (1) de ladite valve d'aspiration (52).

6. Dispositif selon l'une quelconque des revendications précédentes, **caractérisé en ce que** ladite chambre de compression/décompression (Ch) est amovible.

7. Dispositif selon l'une quelconque des revendications précédentes, **caractérisé en ce** ladite chambre de compression/décompression (Ch) est formée dans matériau souple, élastique et non toxique tel que le caoutchouc, et en ce qu'elle a la forme et les couleurs d'une figurine distrayante.

8. Dispositif selon l'une quelconque des revendications précédentes, **caractérisé en ce que** l'ensemble des organes constitutifs dudit dispositif sont ajustés par frottement.

## Claims

1. A device for aspirating nasal mucous, including a body (C) forming a cavity having a first open end (1) and second open end (2), a removable nozzle (E) and a compression/decompression chamber (Ch) in sealed fluid communication with said first and second open ends, said body including two valves (51, 52) of which one (52) is an aspiration valve placed inside said body between said first and second open ends so as to permit air to circulate only from said first open end to said second open end and of which the other (51) is an exhaust valve placed at the periphery of said body, between said aspiration valve and said second open end, so as to permit air to circulate only from inside said body to the outside.

2. A device according to claim 1, **characterized in that** one or the other of the two valves (51; 52) includes a first disc (9) disposed over an orifice (4) formed at the bottom of a bore (5) and on top of which is a first annular part (10) having a plurality of radial grooves (11, 12, 13, 14) on its face intended to face said orifice.

3. A device according to claim 1 or claim 2, **characterized in that** one or the other of said two valves (51; 52) includes a second disc (17) disposed on an annular flange (15) and on top of which is a second annular part (18) having, at the periphery of its face intended to face said annular flange, a plurality of teeth (19, 20, 21, 22) at the base of which small projections (30, 31, 32, 33) extend radially toward the center of said second annular part.

4. A device according to any preceding claim, **characterized in that** a strainer (Cr) having a first end (35) closed by a part (36) provided with a plurality of small orifices (36') and an open second end (37) is placed in said body (C) between said first opening (1) and said aspiration valve (52), said second open end facing said aspiration valve.

5. A device according to claim 4, **characterized in that** said strainer (Cr) includes an extractor rod (38) fixed rigidly to said part (36) and **in that** the length of said rod is more or less equal to the distance between said first end (1) and said aspiration valve (52).

6. A device according to any preceding claim, **characterized in that** said compression/decompression chamber (Ch) is removable.

7. A device according to any preceding claim, **characterized in that** said compression/decompression chamber (Ch) is formed from a flexible, elastic and nontoxic material such as rubber and **in that** it has the shape and the colors of an amusing figurine.

8. A device according to any preceding claim, **characterized in that** all the components of said device are assembled by means of a friction fit.

## Patentansprüche

1. Vorrichtung zum Absaugen von Nasenschleim, bestehend aus einem einen Hohlraum bildenden Körper (C), der ein erstes (1) und ein zweites (2) offenes Ende aufweist, einem abnehmbaren Aufsatz (E) und einer Kompressions/Dekompressions-Kammer (Ch), die mit dem ersten bzw. mit dem zweiten offenen Ende in fluiddichter Verbindung ist, wobei der Körper zwei Ventile (51, 52) aufweist, von denen das eine (52) ein Saugventil, das im Inneren des Körpers zwischen dem ersten und dem zweiten offenen Ende so angeordnet ist, dass es nur eine Luftströmung von dem ersten offenen Ende zum zweiten offenen Ende zulässt, und das andere (51) Auslassventil ist, das am Umfang dieses Körpers zwischen dem Saugventil und dem zweiten offenen Ende so angeordnet ist, dass es nur eine Luftströmung aus dem Inneren des Körpers nach außen zulässt.

2. Vorrichtung nach Anspruch 1, **dadurch gekennzeichnet, dass** das eine oder das andere der beiden Ventile (51; 52) ein erstes Plättchen (9) aufweist, das auf eine am Boden einer Bohrung (5) gebildete Öffnung (4) aufgelegt ist und auf welches ein erstes ringförmiges Teil (10) aufgesetzt ist, das auf seiner Seite, die dieser Öffnung zugewandt angeordnet sein soll, mehrere radiale Nuten (11, 12, 13, 14) aufweist.

3. Vorrichtung nach Anspruch 1 oder 2, **dadurch gekennzeichnet, dass** das eine oder das andere der beiden Ventile (51; 52) ein zweites Plättchen (17) aufweist, das auf einen ringförmigen Kragen (15) aufgelegt ist und auf das ein zweites ringförmiges Teil (18) aufgesetzt ist, das am Umfang seiner Fläche, die diesem ringförmigen Kragen zugewandt angeordnet sein soll, mehrere Zähne (19, 20, 21, 22) aufweist, an deren Basis kleine Erhebungen (30, 31, 32, 33) sich radial auf den Mittelpunkt des zweiten ringförmigen Teils zu erstrecken.

4. Vorrichtung nach einem der vorhergehenden Ansprüche, **dadurch gekennzeichnet, dass** in dem Körper (C) zwischen der ersten Öffnung (1) und dem Saugventil (52) ein Saugkorb (Cr) angeordnet ist, der ein erstes Ende (35), das durch eine mit einer Vielzahl von kleinen Öffnungen (36') versehene Wand (36) geschlossen ist, und ein zweites offenes Ende (37) aufweist, das gegenüber dem Saugventil angeordnet ist.

5. Vorrichtung nach Anspruch 4, **dadurch gekennzeichnet, dass** der Saugkorb (Cr) einen Extraktionsstab (38) aufweist, der an der Wand (36) starr befestigt ist, und dass die Länge dieses Stabs ungefähr gleich dem Abstand des ersten Endes (1) von dem Saugventil (52) ist.

6. Vorrichtung nach einem der vorhergehenden Ansprüche, **dadurch gekennzeichnet, dass** die Kompressions/Dekompressions-Kammer (Ch) abnehmbar ist.

7. Vorrichtung nach einem der vorhergehenden Ansprüche, **dadurch gekennzeichnet, dass** die Kompressions/Dekompressions-Kammer (Ch) aus einem biegsamen, elastischen nichtgiftigen Werkstoff, wie Kautschuk, hergestellt ist, und dass sie die Form einer Unterhaltungsfigur hat.

8. Vorrichtung nach einem der vorhergehenden Ansprüche, **dadurch gekennzeichnet, dass** alle Bestandteile der Vorrichtung durch Reibung eingepasst sind.
